# EUROPÄISCHE PATENTSCHRIFT

(11) **EP 1 687 038 B1**
(45) Veröffentlichungstag und Bekanntmachung des Hinweises auf die Patenterteilung: **03.07.2013**
(21) Anmeldenummer: 04765631.9
(22) Anmeldetag: 27.09.2004
(51) Int. Cl.: A61L 15/60, A61F 13/531

(54) **ABSORBIERENDER HYGIENEARTIKEL**
ABSORBENT SANITARY PRODUCT
ARTICLE HYGIENIQUE ABSORBANT

(30) Priorität: 29.11.2003 DE 10355919
(43) Veröffentlichungstag der Anmeldung: 09.08.2006
(73) Patentinhaber: Paul Hartmann AG, 89522 Heidenheim (DE)
(72) Erfinder: ZOCH, Matthias, 89555 Steinheim (DE); OSTERTAG, Wolfgang, 89547 Gerstetten/Dettingen (DE)
(74) Vertreter: Friz, Oliver
(86) Internationale Anmeldenummer: PCT/EP2004/010804
(87) Internationale Veröffentlichungsnummer: WO 2005/051439

(56) Entgegenhaltungen:
- EP-A- 0 080 382
- WO-A1-03/052190
- US-A- 4 429 001
- US-A- 4 531 945
- US-A- 4 604 313

## Beschreibung

Die vorliegende Erfindung betrifft einen Körperflüssigkeiten absorbierenden Hygieneartikel zur einmaligen Anwendung, wie Windel, Inkontinenzvorlage, Damenbinde oder Slipeinlage, umfassend ein zumindest bereichsweise flüssigkeitsdurchlässiges Deckblatt, ein zumindest bereichsweise flüssigkeitsdichtes-Rückenblatt und einen zwischen Deckblatt und Rückenblatt angeordneten Saugkörper, wobei der Saugkörper eine der dauerhaften Speicherung von Körperflüssigkeit dienende erste Speicherschicht umfasst, welche hydrophilisierte schmelzgeblasene Mikrofasern und partikelförmiges superabsorbierendes Material umfasst.

Saugkörper mit partikelförmigen superabsorbierenden Materialien und schmelzgeblasenen, im sogenannten "Meltblown-Verfahren" hergestellten Fasern sind bereits bekannt geworden. So offenbart EP 0 080 382 B1 einen Hygieneartikel, dessen Saugkörper nach einer Ausführungsform 13 Gew.-% und nach einer anderen Ausführungsform 17 Gew.-% partikelförmiges superabsorbierendes Material aufweist, welches in einem dreidimensionalen Fasernetzwerk von schmelzgeblasenen Mikrofasern aufgenommen und darin weitgehend immobilisiert ist. Nach der Offenbarung der EP 0 080 382 B1 weisen die schmelzgeblasenen Mikrofasern vorzugsweise einen Durchmesser von 1-50 µm auf.

EP 0 159 630 A2 offenbart einen Hygieneartikel, dessen Saugkörper eine erste Speicherschicht aufweist, die eine Mischung aus schmelzgeblasenen Mikrofasern, Zellulosefasern und superabsorbierendem Material aufweist. Der Gehalt an superabsorbierendem Material bezogen auf die Masse dieser Speicherschicht liegt zwischen 20 und 60, vorzugsweise zwischen 5 und 22 Gew.-%.

WO 03/052190 A1 offenbart eine Saugkörperstruktur zur Verwendung in einem Hygieneartikel mit thermoplastischen Mehrkomponentenfasern, bei denen es sich um schmelzgeblasene Mikrofasern handelt, und mit partikelförmigem super absorbierendem Material, welches darin aufgenommen ist. Der Anteil des superabsorbierenden Materials beträgt generell 5 bis 90 Gew.-%, insbesondere 10 bis 60 Gew.-% und vorzugsweise 20 bis 50 Gew.-%. Die Druckschrift lehrt, dass die schmelzgeblasenen Mikrofasern in derart schmelzflüssigem Zustand mit dem partikelförmigen superabsorbierenden Material vermischt werden sollen, dass sich eine klebende Verbindung zwischen Mikrofasern und partikelförmigem superabsorbierendem Material ausbildet. Hierdurch soll eine hinreichende Festigkeit des Vliesstoffmaterials erreicht werden. Nach der Lehre dieser Druckschrift soll außer den thermoplastischen Mikrofasern ein beträchtlicher Anteil zellulosischer Fasern ("pulp fibers") von 5 bis 97, vorzugsweise 35 bis 95 Gew.-%, vorhanden sein. Konkrete Ausführungsbeispiele sind aber nicht offenbart.

In ähnlicher Weise offenbart WO 03/052191 A1 einen Coform-Vliesstoff mit schmelzgeblasenen Mehrkomponentenfasern und einem zweiten Material, das auch superabsorbierende Partikel umfassen oder aus solchen bestehen kann. Aus der Druckschrift ergibt sich eine unüberschaubare Vielfalt von Bereichen der jeweiligen Bestandteile, die bei den Mehrkomponentenfasern 1 bis 95 Gew.-%, insbesondere 2 bis 50 Gew.-%, insbesondere 5 bis 30 Gew.-% und bei dem partikelförmigen superabsorbierenden Material 5 bis 90, insbesondere 10 bis 60 und vorzugsweise 20 bis 50 Gew.-% beträgt. Auch in dieser Druckschrift sind aber keine konkreten Materialzusammensetzungen angegeben. Es findet sich noch der Hinweis, dass die schmelzgeblasenen Fasern eine Haftverbindung mit dem zweiten Material, welches partikelförmiges superabsorbierendes Material umfassen kann, eingehen.

US-A-4,429,001 offenbart einen faserbasierten Schichtwerkstoff, der auch bei Hygieneartikeln Verwendung finden kann. Bei diesem Schichtwerkstoff bildet ein Wirrfasergelege aus schmelzgeblasenen Mikrofasern eine Matrix für superabsorbierende Partikel, die darin gehalten sind. Schmelzbindungen sind nicht angesprochen. Der gewichtsprozentuale Anteil der Partikel beträgt zwischen 30 und 85 Gew.-%.

Der vorliegenden Erfindung liegt die Aufgabe zu Grunde, bei einem absorbierenden Hygieneartikel mit einer Speicherschicht, die hydrophilisierte schmelzgeblasene Mikrofasern und partikelförmiges superabsorbierendes Material umfasst, noch weitgehender zu erreichen, dass das partikelförmige superabsorbierende Material im trockenen Zustand der Speicherschicht darin immobilisiert ist und dass die Speicherschicht aber zugleich eine bessere Nassfestigkeit aufweist, als dies bislang erreicht worden ist.

Diese Aufgabe wird erfindungsgemäß gelöst durch einen Hygieneartikel mit den Merkmalen des Anspruchs 1.

Mit der vorliegenden Erfindung wird der Vorschlag unterbreitet, eine Speicherschicht bei einem Hygieneartikel mit sehr hohem gewichtsprozentualem Anteil an partikelförmigem superabsorbierendem Material zu schaffen, welches in einem dreidimensionalen Netzwerk von schmelzgeblasenen Mikrofasern aufgenommen ist. Das dreidimensionale Netzwerk ist durch eine Vielzahl von Schmelzbindungen zwischen den Mikrofasern stabilisiert. Diese werden gebildet, wenn die Mikrofasern quasi in statu nascendi miteinander verwirbelt werden und dabei eine Vielzahl von Kreuzungspunkten oder Überschneidungen der Mikrofaserstränge miteinander gebildet werden, die an diesen Stellen sofort zumindest oberflächenmäßig miteinander verschmelzen. Beim Zusammenbringen der schmelzgeblasenen Mikrofasern mit dem partikelförmigen superabsorbierenden Material sind die Mikrofasern nach der vorliegenden Erfindung aber schon derart erkaltet bzw. erstarrt, dass es zwischen den Mikrofasern und dem partikelförmigen superabsorbierenden Material, die in der Reel nicht thermisch kompatibel sind, nicht oder nur in geringem Umfang zur Ausbildung von Haftverbindungen kommt. Das partikelförmige superabsorbierende Material ist also mit den Fasern des dreidimensionalen Netzwerks überwiegend nicht klebend verbunden, sondern es ist darin eher mechanisch eingeschlossen. Wenn die so gebildete Speicherschicht eingenässt wird und das partikelförmige superabsorbierende Material zu quellen beginnt bzw. in den Gelzustand übergeht, so wird hierdurch das dreidimensionale Netzwerk nicht zerstört, sondern das superabsorbierende Material durchdringt quasi die dreidimensionale Netzwerkstruktur mit der Folge, dass deren Festigkeit durch die Einnässung nicht so stark beeinträchtigt wird, wie dies bei bekannten Saugkörperschichten und Saugkörperstrukturen der Fall ist. Die Nassfestigkeit der Speicherschicht in Maschinenrichtung beträgt nämlich zumindest 40 % der Trockenfestigkeit. Insofern wird auf die weiter unten erörterte Testmethode hingewiesen.

Wenn im Zusammenhang mit der vorliegenden Erfindung von Mikrofasern die Rede ist, so werden hierunter Fasern mit einem geringen Durchmesser von nicht mehr als 15 µm verstanden. Ein vorteilhafter Durchmesser von Mikrofasern liegt zwischen 1 und 15, vorzugsweise zwischen 1 und 10 und insbesondere zwischen 1 und 8 µm

Um das durch die schmelzgeblasenen Mikrofasern gebildete Fasernetzwerk mit einem ausreichenden Saugvermögen zu versehen, ist vorgesehen die Mikrofasern zu hydrophilisieren. Dies wird vorzugsweise derart verwirklicht, dass dem insbesondere an sich hydrophoben Polymer, aus dem die Mikrofasern gebildet werden, ein Hydrophilisierungsmittel, vorzugsweise in die Polymerschmelze beigemischt wird. Als vorteilhaft haben sich insbesondere Alkylsulfonate, Fettsäurederivate oder Flourchemikalien wie sie in der Veröffentlichung "Polymer Melt Additives: Their Chemistry, Structure and Uses" (Autoren Gasper et al. Vortrag während der Insight 1999 - Nonwoven Business/Fiber & Fabric Conferences, San Diego, California, 1-2 November 1999, Proceedings herausgegeben durch Marketing Technology Services, Inc.) beschrieben sind. Ein besonders vorteilhaftes Hydrophilisierungsmittel ist unter dem Handelsnamen Igasurf HL 560 bei der Fa. CIBA Specialty Chemicals, Basel (CH),zu beziehen. Hinsichtlich der eingesetzten Mengen an Hydrophilisierungsmittel sind 1-15%, insbesondere 2-8% berechnet als Gewichtsanteil am Gesamtgewicht des Polymers vorteilhaft.

Es hat sich als besonders vorteilhaft erwiesen, wenn die durchschnittliche Größe (Massenmittelwert) des partikelförmigen superabsorbierenden Materials zwischen 100 und 800 µm, insbesondere zwischen 200 und 700 µm liegt, ermittelt nach der in EP 0 304 319 B1 offenbarten Prüfmethode.

In Weiterbildung der Erfindung von besonderer Bedeutung wird die Dicke der ersten Speicherschicht - gemessen bei einem Prüfdruck von 0,5 kPa - so gewählt, dass sie vorzugsweise das 1;5 bis 5-Fache, insbesondere das 1,5 bis 4-Fache, insbesondere das 1,5 bis 3-Fache und weiter insbesondere das 1,5 bis 2,5-Fache der durchschnittlichen Größe der partikelförmigen superabsorbierenden Materialien innerhalb der Speicherschicht beträgt. Es hat sich gezeigt, dass bei dem erfindungsgemäß beanspruchten SAP-Gehalt und Fasergehalt dann ein ausreichendes Porenvolumen zur Flüssigkeitsaufnahme und -leitung einerseits zur Verfügung steht und andererseits die SAP-Partikel derart voneinander beabstandet sind, dass eine gegenseitige Quellungsbehinderung der SAP-Partikel in nur geringem Ausmaß stattfindet. Die Dicke der ersten Speicherschicht ist daher eher gering, wenn man von einer durchschnittlichen Partikelgröße im Bereich von 100 bis 800 µm ausgeht.

Nach einer bevorzugten Ausführungsform vermag die Speicherschicht bei einem Test auf vertikale Flüssigkeitsaufnahme eine Saughöhe von wenigstens 2 cm insbesondere wenigstens 3,5 cm aufzuweisen. Auch hierfür wird auf die weiter unten erörterte Testmethode hingewiesen.

Die erste Speicherschicht kann in vorteilhafter Weise auf einer oder beiden Seiten eine weitere Sauglage aufweisen, die zu einem gewichtsprozentualen Anteil von wenigstens 50, insbesondere wenigstens 60, insbesondere wenigstens 70, insbesondere wenigstens 80 und weiter insbesondere wenigstens 90 Gew.-% schmelzgeblasene Mikrofasern umfasst. Diese Sauglage oder diese Sauglagen bilden dann quasi eine Hüllschicht für die erste Speicherschicht. Ihr Flächengewicht beträgt vorzugsweise 2 bis 10 g/m², insbesondere 2-5 g/m² ist also verhältnismäßig gering. Außerdem erweist es sich als vorteilhaft, wenn der Faserdurchmesser der Mikrofasern dieser zusätzlichen Sauglage oder dieser zusätzlichen Sauglagen geringer ist als der Faserdurchmesser der Mikrofasern der ersten Speicherschicht. Als vorteilhafte Faserdurchmesser der Mikrofasern dieser zusätzlichen Sauglage oder dieser zusätzlichen Sauglagen hat sich eine Größe von 1-10 µm, insbesondere 1-5 µm, vorzugsweise 1-3 µm erwiesen.

Auch die Mikrofasern dieser zusätzlichen Sauglage oder dieser zusätzlichen Sauglagen sind vorzugsweise insbesondere permanent hydrophi lisiert.

Es erweist sich des Weiteren als vorteilhaft, wenn die Mikrofasern der Sauglage oder der Sauglagen zu den Mikrofasern der ersten Speicherschicht thermisch kompatibel, insbesondere aus demselben Polymer gebildet sind. Auf diese Weise können sich Verbindungsstellen zwischen den jeweiligen Fasern ausbilden.

Als Polymer kommen alle thermoplastischen Polymere in Frage, insbesondere Polyolefine wie Polypropylen und Polyethylen, aber auch Polyester wie beispielsweise Polyethylenterephtalat.

Des Weiteren erweist es sich als vorteilhaft, wenn der erfindungsgemäße Hygieneartikel eine offenporige Flüssigkeitsaufnahme- und -verteilerschicht aufweist, die vorzugsweise jedoch nicht notwendigerweise aus Fasern gebildet ist.

### Testmethode zur Nassfestigkeit und Trockenfestigkeit:

Die nachfolgende Testmethode beschreibt ein Verfahren zur Bestimmung der Zugfestigkeit und Bruchdehnung von Saugkörpern oder Saugkörperschichten im trockenen und nassen Zustand. Die ermittelten Werte erlauben eine Beurteilung des Zusammenhalts der Bestandteile der Schicht und geben somit Auskunft über deren Trockenfestigkeit und deren Nassfestigkeit.

Zur Durchführung des Testverfahrens erweist sich ein Zugprüfgerät nach EN ISO 527-1 (vom April 1996) sowie ein rechteckiges Stanzmesser der Größe 50 mm x 150 mm (± 0,25 mm) als vorteilhaft.

Zur Durchführung des Testverfahrens werden aus einem das Probenmaterial bildenden Flachmaterial 5 Probenkörper der Größe 50 mm x 150 mm herausgestanzt, und zwar so, dass die längere Abmessung parallel zur Maschinenrichtung bei der Herstellung der Flachmaterialbahn orientiert ist.

Das 50 mm x 150 mm große Probenstück wird beidseits an seinen Längsenden in eine flächenhaft erstreckte Klemme, deren Breite wenigstens der Breite der Probe, also 50 mm, beträgt, eingespannt, und zwar mit einer Einspannlänge von beidseits jeweils 25 mm, so dass sich eine nicht eingespannte Probenlänge von 100 mm ergibt.

Da die Saugkörperproben bei Flüssigkeitszugabe quellen, erweist sich das Einspannen der gequollenen Bereiche in das Zugprüfgerät als problematisch. Es kommt dabei zu Verquetschungen an den nassen gequollenen Oberflächenbereichen, wodurch der Probenkörper stets an der Einspannstelle reißt. Um dies zu verhindern, soll die Einspannstelle während des Quellens trocken gehalten werden.

Dies wird erreicht, wenn der jeweilige Probenkörper wie vorausgehend beschrieben mit einer praktisch über seine gesamte Breite erstreckten Klemme eingespannt und in diesem eingespannten Zustand eingenässt wird. Das quellende superabsorbierende Material bildet dann nämlich innerhalb des von der Klemme überfangenen Bereichs einen flüssigkeitsdichten Abschluss, was bedeutet, dass der eingespannte Bereich des Probenkörpers wenige Millimeter innerhalb der Klemme trocken bleibt.

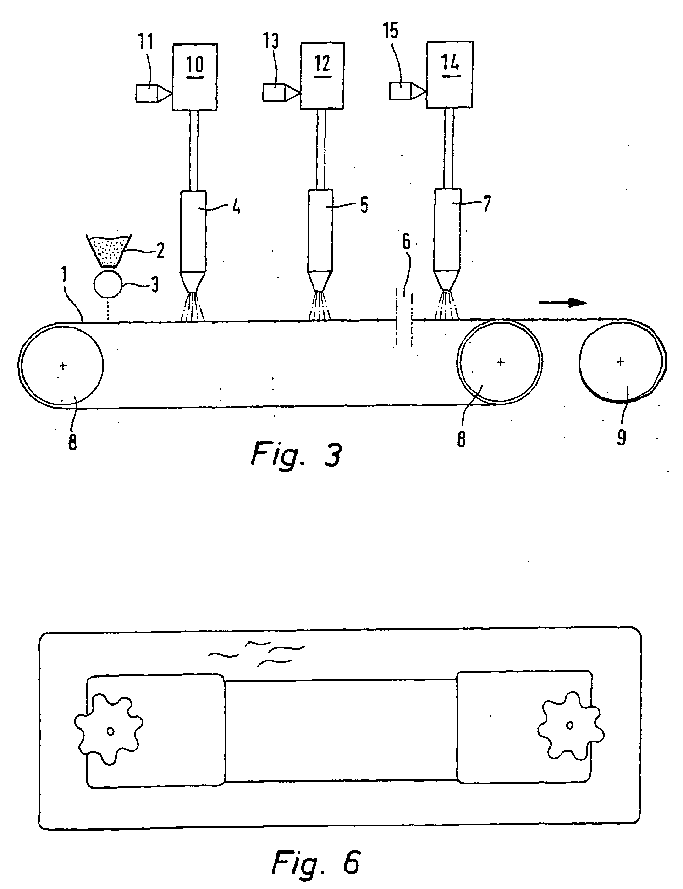

Der wie vorstehend beschrieben eingespannte Probenkörper wird wie in der Figur 6 skizziert in eine Schale mit wässriger Flüssigkeit gelegt. Die Flüssigkeitsmenge ist dabei abhängig von der Menge an superabsorbierendem Material, die in der Saugkörperschicht enthalten ist. Man rechnet mit 15 g Flüssigkeit pro g superabsorbierendem Material. Die Saugkörperprobe verbleibt für drei Minuten in der Schale. Sie wird danach entnommen und zunächst auf einer ebenen Unterlage abgelegt, um die zuvor erwähnten Klemmen abzulösen. Hierbei erkennt man, dass der zuvor eingespannte Bereich des Probenkörpers trocken geblieben ist. Mit diesem trocken gebliebenen Bereich wird nun der Probenkörper in ebenfalls flächenhaft erstreckte Klemmen des Zugprüfgeräts eingespannt. Er wird zunächst mit einem Ende in die obere Klemme des Zugprüfgeräts eingespannt. Die sich ergebende Kraftanzeige, die auf die Gewichtskraft der nassen Probe zurückgeht, muss auf 0 eingestellt werden. Danach wird das untere Ende des Probenkörpers in die untere Klemme spannungsfrei eingespannt. Die Einspannung erfolgt dabei so, dass sich der Übergangsbereich von trockenem Längsende des Probenkörpers zu dem eingenässten Bereich des Probenkörpers außerhalb der eingespannten Zone befindet.

Es werden bei der Prüfung jedes Probenkörpers mindestens n = 5 Einzelmessungen durchgeführt. Die Klemmen des Zugprüfgeräts werden dann mit einer Prüfgeschwindigkeit von 100 mm/min auseinanderbewegt und es wird die sich hierbei ergebende Zugkraft innerhalb des Probenkörpers in Richtung der Prüfbewegung ermittelt. Diejenige Kraft, bei der der Probenkörper reißt, wird als Bruchfestigkeit bezeichnet. Er definiert bei der Durchführung der Messung eines trockenen Probenkörpers dessen Trockenfestigkeit und bei der Messung des wie vorstehend beschrieben eingenässten Probenkörpers dessen Nassfestigkeit.

### Vertikale Flüssigkeitsaufnahme:

Mit diesem Testverfahren wird die Dochtwirkung eines Probenkörpers, d. h. dessen Fähigkeit, Flüssigkeit aufzusaugen und aktiv, also nicht durch Schwerkraft oder dergleichen unterstützt, zu verteilen.

Als Prüfgeräte werden benötigt ein Wasserbehälter, ein Probenhalter mit einem Ständer, eine Präzisionswaage, ein Stanzmesser zum Ausstanzen der Probenkörper aus einer Flachmaterialbahn in Maschinenrichtung und eine Stoppuhr sowie eine Prüflösung, bei der es sich um 0,9 %-ige Lösung von NaCl in demineralisiertem Wasser handelt. Unter Verwendung des Stanzmessers werden Probenkörper einer Abmessung von 10 O mm x 150 mm (± 0,25 mm) aus einer Flachmaterialbahn ausgestanzt. Der so gebildete Probenkörper wird mit einem freien Längsende an dem Probenhalter eingespannt und hängt frei in den zunächst leeren Wasserbehälter hinein. Es wird sodann die Prüflösung vorsichtig in den Wasserbehälter eingefügt, so dass das untere freie Ende des Probenkörpers in die Flüssigkeit eintaucht. Es wird dann nach 20 Minuten die Steighöhe der Flüssigkeit in dem Probenkörper, die auf kapillare Sogwirkung in dem Probenkörper zurückzuführen ist, ermittelt und in Zentimeter angegeben.

Weitere Merkmale, Einzelheiten und Vorteile der Erfindung ergeben sich aus den Patentansprüchen und aus der zeichnerischen Darstellung und nachfolgenden Beschreibung des erfindungsgemäßen Hygieneartikels. In der Zeichnung zeigt:
- Figur 1: eine schematische Schnittansicht einer Speicherschicht eines erfindungsgemäßen Hygieneartikels;
- Figur 2: eine maßstabsgetreue Skizze der Einbindung eines Partikels aus superabsorbierendem Material in eine dreidimensionale Netzwerkstruktur aus Mikrofasern;
- Figur 3: eine schematische Darstellung einer Faserverbundwerkstoffherstellungsanlage;
- Figur 4: eine Schnittansicht einer Saugkörperschicht mit einer Deckschicht;.
- Figur 5: eine Schnittansicht einer Saugkörperschicht mit Deckschichten auf beiden Seiten und
- Figur 6: einen eingespannten Probenkörper beim Einnässen.

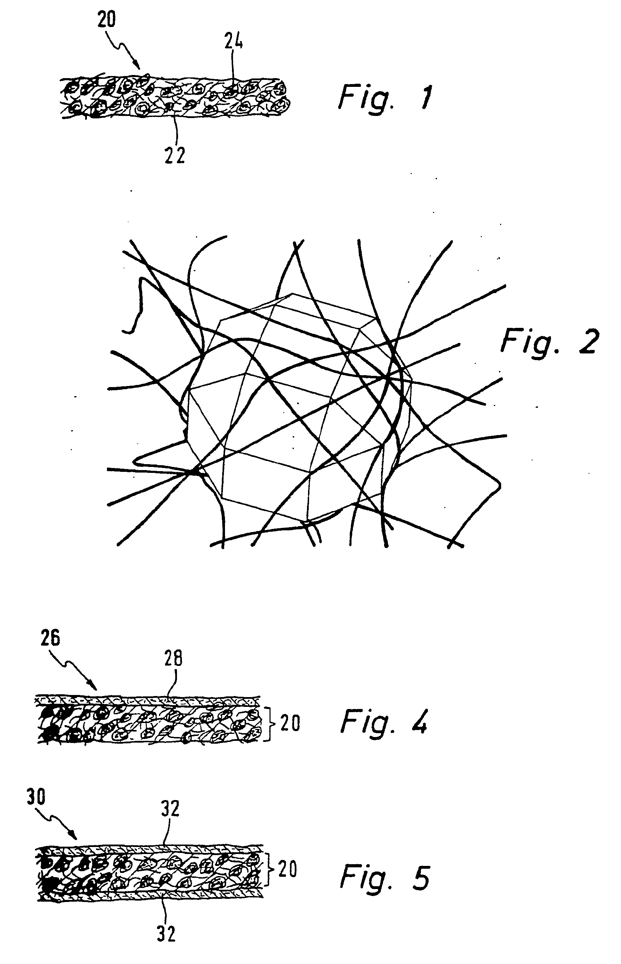

Figur 1 zeigt eine Schnittansicht einer erfindungsgemäßen Speicherschicht 20. Die Speicherschicht 20 umfasst hydrophilisierte Polypropylenmeltblownfasern 22 mit einem Faserdurchmesser von 3-6 µm. Die flächenbezogene Masse des Faseranteils beträgt 20 g/m². Die Speicherschicht 20 weist außerdem SAP-Partikel 24 einer durchschnittlichen Partikelgröße (Massenmittelwert) von ca. 400 µm auf. Die flächenbezogene Masse der SAP-Partikel 24 beträgt 220 g/m². Der gewichtsbezogene SAP-Anteil beträgt somit 91,5%. Die Dicke der Speicherschicht beträgt 1,2 mm.

Die Saughöhe der Speicherschicht 20 nach der oben beschriebenen Testmethode beträgt, 3,5 cm. Die Zugfestigkeittrocken dieser Speicherschicht 20 wurde nach der oben beschriebenen Testmethode mit 6.1 N bestimmt. Die Zugfestigkeit-nass nach der oben beschriebenen Testmethode betrug 3,6 N.

Figur 2 zeigt vergrößert eine schematische jedoch maßstabsgerechte Darstellung eines SAP-Partikels 24 einer Größe von 450 µm, welcher in ein dreidimensionales Netzwerk von Meltbownfasern 22 eingebunden ist.

Die Speicherschicht 20 wurde hergestellt mittels einer in Figur 3 dargestellten Faserverbundwerkstoffherstellungsanlage. Die SAP Partikel wurden über das Feststoffaufgabemittel 2 und die Streuwalze 3 auf ein sich in Pfeilrichtung kontinuierlich bewegendes Ablegeband 1 aus Teflon dosiert. Auf diese SAP Partikelschicht wurde über eine Meltblownfaserbildungseinheit, welche eine Meltblownpolymerdosierungsvorrichtung 10, eine Zudosierungsvorrichtung 11 für Additive wie Hydrophilisierungsmittel und eine Meltblowndüsenvorrichtung 4 umfasst ein die Meltblownfasern enthaltender Luftstrom gerichtet, derart, dass auf dem Ablegeband eine Verwirbelung und damit einhergehend keine Schichtung, sondern eine weitestgehend homogene Mischung der SAP-Partikel und der Meltblownfasern erfolgte. Temperatur und Geschwindigkeit des die Meltblownfasern enthaltenden Luftstroms wurden so gewählt, dass nur in geringem Ausmaß Schmelzbindungen zwischen SAP-Partikeln und Meltblownfasern entstanden. Die weiteren Meltblownfaserbildungseinheiten (5, 12, 13 und 6, 7, 14, 15) waren außer Funktion gesetzt.

Es versteht sich, dass die Vermischung von SAP-Partikeln und Meltblownfasern auch abweichend vom zuvor dargestellten Verfahren etwa dadurch bewirkt werden kann, dass die SAP-Partikel direkt in den die Metlblownfasern enthaltenden Luftstrom dosiert werden können und die sich dadurch ergebende Faser/SAP-Mischung anschließend auf das Band zur Bildung der erfindungsgemäßen Speicherschicht gelegt wird, wie dies etwa die oben zitierten EP 0 159 630 A2 oder die WO 03/052190 zeigen. Durch Steuerung insbesondere der Temperatur des Meltblownfasern enthaltenden Luftstroms vor und nach der Zudosierung der SAP-Partikel kann das Ausmaß und die Anzahl der Schmelzbindungen zwischen SAP-Partikeln und Meltblownfasern gezielt beeinflusst werden. Je geringer nämlich die Temperatur der Meltblownfasern zum Zeitpunkt der Berührung mit SAP-Partikeln ist, desto weniger Schmelzbildungen zu den SAP-Partikeln werden erzielt, während Schmelzbildungen der Fasern untereinander, das heißt innerhalb thermisch kompatiblen Materials, auch bei geringeren Temperaturen noch auftreten. Dies natürlich nur insoweit die Meltblownfasern nicht völlig erkaltet und damit erstarrt sind, sondern noch eine gewisse Restklebrigkeit ("tackiness") aufweisen.

In einer weiteren erfindungsgemäßen Ausführungsform der Speicherschicht nach Figur 1 umfasst die Speicherschicht wiederum hydrophilisierte Polypropylenmeltblownfasern mit einem Faserdurchmesser von 3-6 µm. Die flächenbezogene Masse des Faseranteils beträgt 13 g/m². Die Speicherschicht weist außerdem SAP-Partikel einer durchschnittlichen Partikelgröße von ca. 350 µm auf. Die flächenbezogene Masse der SAP-Partikel beträgt 220 g/m². Der gewichtsbezogene SAP-Anteil beträgt somit 93,6%. Die Dicke der Speicherschicht beträgt 1,0 mm.

Figur 4 zeigt eine Schnittansicht einer vorteilhaften Weiterbildung der Saugkörperschicht. Die Speicherschicht 26 weist einseitig als weitere Saugschicht eine insbesondere SAP-freie Meltblownfaserschicht 28 auf. Der Durchmesser der Fasern dieser Schicht beträgt 2-3µm. Das Flächengewicht beträgt lediglich 4 g/m². Hierfür wird auf die oben beschriebene erfindungsgemäße Speicherschicht direkt mittels einer nachfolgenden weiteren Meltblownfaserbildungseinheit (5, 12, 13) eine dünne Schicht Meltblownfasern aufgebracht wird, so dass diese Meltblownfaserschicht und die Saugkörperschicht eine haftende Bindung eingehen. Die in Figur 3 dargestellte dritte Meltblownfaserbildungseinheit (6, 7, 14, 15) ist außer Funktion gesetzt.

In einer weiteren vorteilhaften Weiterbildung der Saugkörperschicht weist die Speicherschicht 30 beidseitig Saugschichten, insbesondere SAP-freie Meltblownfaserschichten 32 auf (Figur 5). Diese Weiterbildung der Saugkörperschicht lässt sich herstellen, indem auf die erste Oberfläche der oben beschriebenen erfindungsgemäßen Speicherschicht 22 direkt mittels einer nachfolgenden weiteren Meltblownfaserbildungseinheit (5, 12, 13) zunächst wie zuvor beschrieben eine erste dünne Schicht Meltblownfasern aufgebracht wird. Anschließend wird dieser Schichtverbund mittels üblicher Verfahren gewendet (Bezugszeichen 6), und nachfolgend wird auf die zweite Oberfläche der Speicherschicht direkt mittels einer nachfolgenden weiteren Meltblownfaserbildungseinheit (7, 14, 15) analog eine zweite dünne Schicht Meltblownfasern aufgebracht.

Die Vorteile der ein- oder beidseitig meltblownfasernbeschichteten erfindungsgemäßen Speicherschichten sind eine bessere Flüssigkeits verteilerkapazität, höhere Nass- und Trockenfestigkeiten sowie die wirksame Verhinderung des Austretens von SAP-Partikeln aus der Speicherschicht.

## Patentansprüche

1. Körperflüssigkeiten absorbierender Hygieneartikel zur einmaligen Anwendung, wie Windel, Inkontinenzvorlage, Damenbinde oder Slipeinlage, umfassend ein zumindest bereichsweise flüssigkeitsdurchlässiges Deckblatt ein zumindest bereichsweise flüssigkeitsdichtes Rückenblatt und einen zwischen Deck- und Rückenblatt angeordneten Saugkörper, wobei der Saugkörper eine der dauerhaften Speicherung von Körperflüssigkeit dienende erste Speicherschicht (20, 26, 30) umfasst, welche zu 5-30 Gew.-% hydrophilisierte schmelzgeblasene Mikrofasern (22), zu 70-95 Gew.-% partikelförmiges superabsorbierendes Material (24) und gegebenenfalls bis zu höchstens 10 Gew.-% einer weiteren partikel- oder faserförmigen Komponente aufweist, wobei das Flächengewicht der schmelzgeblasenen Mikrofasern (22) 6-25 g/m² beträgt, wobei die schmelzgeblasenen Mikrofasern (22) untereinander durch eine Vielzahl von Schmelzbindungen nassfest verbunden sind, derart, dass die schmelzgeblasenen Mikrofasern (22) ein dichtes, das partikelförmige superabsorbierende Material (24) umschließendes und immobilisierendes und dreidimensionales Netzwerk bilden, und wobei zwischen den schmelzgeblasenen Mikrofasern (22) und dem partikelförmigen superabsorbierenden Material (24) keine oder nur in geringem Ausmaß Schmelzbindungen vorgesehen sind, und dass die Speicherschicht (20, 26, 30) eine Nassfestigkeit gemessen in Maschinenrichtung aufweist, die zumindest 40% der Trockenfestigkeit beträgt.

2. Hygieneartikel nach Anspruch 1, **dadurch gekennzeichnet, dass** die durchschnittliche Größe des partikelförmigen superabsorbierenden Materials D_{SAP} 100-800 µm beträgt und die Dicke der ersten Speicherschicht D_{ISP} zwischen D_{SAP} * 1, 5 und D_{SAP} * 5, insbesondere zwischen D_{SAP} * 1, 5 und D_{SAP} * 4, insbesondere zwischen D_{SAP} * 1,5 und D_{SAP} * 3 und weiter insbesondere zwischen D_{SAP} * 1,5 und D_{SAP} * 2,5 beträgt.

3. Hygieneartikel nach einem der vorherigen Ansprüche, **dadurch gekennzeichnet, dass** die Saughöhe der ersten Speicherschicht (20, 26, 30) mindestens 2 cm, insbesondere mindestens 3 cm, insbesondere mindestens 4 cm, insbesondere mindestens 5 cm und weiter insbesondere. mindestens 6 cm beträgt.

4. Hygieneartikel nach einem der vorstehenden Ansprüche, **dadurch gekennzeichnet, dass** die erste Speicherschicht (26) eine dem Rückenblatt zugewandte Sauglage (28) aufweist, welche zu 100-50 Gew.-%, insbesondere zu 100 - 60 Gew.-%, insbesondere zu 100 - 70 Gew.-%, insbesondere zu 100 - 80 Gew.-% und weiter insbesondere zu 100 - 90 Gew.-% schmelzgeblasene Mikrofasern aufweist.

5. Hygieneartikel nach einem der vorherigen Ansprüche, **dadurch gekennzeichnet, dass** die erste Speicherschicht (26) eine dem Deckblatt zugewandte Sauglage (28) aufweist, welche zu 100-50 Gew.-%, insbesondere zu 100 - 60 Gew.-%, insbesondere zu 100 - 70 Gew.-%, insbesondere zu 100 - 80 Gew.-% und weiter insbesondere zu 100 - 90 Gew.-% schmelzgeblasene Mikrofasern aufweist.

6. Hygieneartikel nach einem der Ansprüche 4 oder 5, **dadurch gekennzeichnet, dass** das Flächengewicht der Sauglage (28, 32) 2-10 g/m², insbesondere 2 - 5 g/m² beträgt und der Faserdurchmesser der schmelzgeblasenen Mikrofasern der Sauglage (28, 32) geringer ist als der Faserdurchmesser der schmelzgeblasenen Mikrofasern (22) der ersten Speicherschicht (20).

7. Hygieneartikel nach einem der Ansprüche 4 bis 6, **dadurch gekennzeichnet, dass** die schmelzgeblasenen Mikrofasern der Sauglage (28, 32) zu den schmelzgeblasenen Mirkofasern (22) der ersten Speicherschicht (20) thermisch kompatibel sind.

8. Hygieneartikel nach einem der vorherigen Ansprüche, **dadurch gekennzeichnet, dass** zwischen der ersten Speicherschicht (20) und dem Deckblatt eine der schnellen Flüssigkeitsaufnahme dienende poröse, vorzugweise faserige Schicht angeordnet ist.

9. Hygieneartikel nach einem der vorherigen Ansprüche, **dadurch gekennzeichnet, dass** die Nassfestigkeit zumindest 50%, insbesondere zumindest 60%, besonders bevorzugt zumindest 70%, ganz besonders bevorzugt zumindest 80% und insbesondere besonders bevorzugt zumindest 90% der Trockenfestigkeit beträgt.

10. Hygieneartikel nach einem der vorherigen Ansprüche, **dadurch gekennzeichnet, dass** die Speicherschicht aus schmelzgeblasenen Mikrofasern und partikelförmigem superabsorbierenden Material besteht.

## Claims

1. Disposable sanitary product for absorbing body liquids such as diaper, incontinence pad, sanitary towel or panty liner, comprising a top sheet at least sections of which are permeable to liquid, a back sheet at least sections of which are impermeable to liquid and an absorbent body disposed between the top sheet and the back sheet, the absorbent body comprising a first storage layer (20, 26, 30) for permanently storing body liquids, which comprises 5 to 30 weight % of hydrophilic melt-blown microfibers (22), 70 to 95 weight % of particulate superabsorbing material (24) and optionally up to maximally 10 weight % of a further particulate or fibrous component, wherein the mass per unit area of the melt-blown microfibers (22) is 6 to 25 g/m², wherein the melt-blown microfibers (22) are connected to each other by a plurality of melt connections to ensure stability in the wet state and in such a manner that the melt-blown microfibers (22) form a dense three-dimensional network which surrounds and immobilizes the particulate superabsorbing material (24), and wherein no or only a few melt connections are provided between the melt-blown microfibers (22) and the particulate superabsorbing material (24), and the storage layer (20, 26, 30) has a strength in the wet state, measured in the machine direction, of at least 40% of the strength in the dry state.

2. Sanitary product according to claim 1, **characterized in that** the average size of the particulate superabsorbing material D_{SAP} is 100 to 800 µm and the thickness of the first storage layer D_{ISP} is between D_{SAP} * 1.5 and D_{SAP} * 5, in particular, between D_{SAP} * 1.5 and D_{SAP} * 4, in particular between D_{SAP} * 1.5 and D_{SAP} * 3, and preferentially between D_{SAP} * 1.5 and D_{SAP} * 2.5.

3. Sanitary product according to any one of the preceding claims, **characterized in that** the absorption level of the first storage layer (20, 26, 30) is at least 2 cm, in particular at least 3 cm, in particular at least 4 cm, in particular, at least 5 cm and moreover in particular at least 6 cm.

4. Sanitary product according to any one of the preceding claims, **characterized in that** the first storage layer (26) has an absorbent layer (28) facing the back sheet, which comprises melt-blown microfibers of an amount of 100 to 50 weight %, in particular 100 to 60 weight %, in particular 100 to 70 weight %, in particular 100 to 80 weight % and moreover, in particular, 100 to 90 weight %.

5. Sanitary product according to any one of the preceding claims, **characterized in that** the first storage layer (26) has an absorbent layer (28) facing the top sheet, which comprises melt-blown microfibers in an amount of 100 to 50 weight %, in particular 100 to 60 weight %, in particular 100 to 70 weight %, in particular 100 to 80 weight % and moreover, in particular, 100 to 90 weight %.

6. Sanitary product according to any one of the claims 4 or 5, **characterized in that** the mass per unit area of the absorbent layer (28, 32) is 2 to 10 g/m², in particular 2 to 5 g/m², and the fiber diameter of the melt-blown microfibers of the absorbent layer (28, 32) is smaller than the fiber diameter of the melt-blown microfibers (22) of the first storage layer (20).

7. Sanitary product according to any one of the claims 4 through 6, **characterized in that** the melt-blown microfibers of the absorbent layer (28, 32) are thermally compatible with the melt-blown microfibers (22) of the first storage layer (20).

8. Sanitary product according to any one of the preceding claims, **characterized in that** a porous preferably fibrous layer is disposed between the first storage layer (20) and the top sheet, which rapidly absorbs liquid.

9. Sanitary product according to any one of the preceding claims, **characterized in that** the strength in the wet state is at least 50%, in particular, at least 60%, with particular preference at least 70%, with particular advantage at least 80% and preferentially at least 90% of the strength in the dry state.

10. Sanitary product according to any one of the preceding claims, **characterized in that** the storage layer consists of melt-blown microfibers and particulate superabsorbing material.

## Revendications

1. Article hygiénique à usage unique destiné à absorber des liquides corporels, comme une couche-culotte, une protection pour l'incontinence, une serviette hygiénique ou un protège-slip, comprenant un film supérieur perméable aux liquides au moins par endroits, un film inférieur imperméable aux liquides au moins par endroits, et un corps absorbant placé entre le film inférieur et le film supérieur, le corps absorbant comportant une première couche de rétention (20, 26, 30) servant à retenir de manière durable un liquide corporel, cette couche étant composée pour 5-30 % en poids de microfibres (22) hydrophilisées soufflées par fusion, pour 70-95 % en poids de matière particulaire superabsorbante (24) et pour éventuellement jusqu'à 10 % en poids d'un autre composant particulaire ou fibreux, le grammage des microfibres (22) soufflées par fusion atteignant 6-25 g/m2, les microfibres (22) soufflées par fusion étant reliées entre elles de manière à résister au mouillage par une pluralité de liaisons par fusion, de sorte que les microfibres (22) soufflées par fusion forment un réseau tridimensionnel dense entourant et immobilisant la matière particulaire superabsorbante (24), et pas ou peu de liaisons par fusion étant présentes entre les microfibres (22) soufflées par fusion et la matière particulaire superabsorbante (24), et si bien que la couche de rétention (20, 26, 28) présente une résistance à l'humidité, mesurée dans le sens de la machine, atteignant au moins 40 % de la résistance à sec.

2. Article hygiénique selon la revendication 1, **caractérisé en ce que** la taille moyenne de la matière particulaire superabsorbante atteint D_{SAP} 100-800 µm et l'épaisseur de la première couche de rétention D_{ISP} est comprise entre D_{SAP} * 1,5 et D_{SAP} * 5, en particulier entre D_{SAP} * 1,5 et D_{SAP} * 4, en particulier entre D_{SAP} * 1, 5 et D_{SAP} * 3 et plus particulièrement entre D_{SAP} * 1, 5 et D_{SAP} * 2,5.

3. Article hygiénique selon l'une quelconque des revendications précédentes, **caractérisé en ce que** la hauteur d'absorption de la première couche de rétention (20, 26, 30) atteint au moins 2 cm, en particulier au moins 3 cm, en particulier au moins 4 cm, en particulier au moins 5 cm et plus particulièrement au moins 6 cm.

4. Article hygiénique selon l'une quelconque des revendications précédentes, **caractérisé en ce que** la première couche de rétention (26) comprend une couche d'absorption (28) tournée vers le film inférieur, ladite couche d'absorption étant composée pour 100-50% en poids, en particulier pour 100-60 % en poids, en particulier pour 100-70 % en poids, en particulier pour 100-80 % en poids et plus particulièrement pour 100-90 % en poids de microfibres soufflées par fusion.

5. Article hygiénique selon l'une quelconque des revendications précédentes, **caractérisé en ce que** la première couche de rétention (26) comprend une couche d'absorption (28) tournée vers le film supérieur, ladite couche d'absorption étant composée pour 100-50 % en poids, en particulier pour 100-60 % en poids, en particulier pour 100-70 % en poids, en particulier pour 100-80 % en poids et plus particulièrement pour 100-90 % en poids de microfibres soufflées par fusion.

6. Article hygiénique selon l'une quelconque des revendications 4 ou 5, **caractérisé en ce que** le grammage de la couche d'absorption (28, 32) atteint 2-10 g/m², en particulier 2-5 g/m² et le diamètre des microfibres soufflées par fusion de la couche d'absorption (28, 32) est inférieur au diamètre des microfibres (22) soufflées par fusion de la première couche de rétention (20).

7. Article hygiénique selon l'une quelconque des revendications 4 à 6, **caractérisé en ce que** les microfibres soufflées par fusion de la couche d'absorption (28, 32) et les microfibres (22) soufflées par fusion de la première couche de rétention (20) sont thermiquement compatibles.

8. Article hygiénique selon l'une quelconque des revendications précédentes, **caractérisé en ce qu'**une couche poreuse, de préférence fibreuse, servant à absorber rapidement les liquides, est disposée entre la première couche de rétention (20) et le film supérieur.

9. Article hygiénique selon l'une quelconque des revendications précédentes, **caractérisé en ce que** la résistance à l'humidité atteint au moins 50 %, en particulier au moins 60 %, de préférence encore au moins 70 %, idéalement au moins 80 % et d'une manière particulièrement préférable au moins 90 % de la résistance à sec.

10. Article hygiénique selon l'une quelconque des revendications précédentes, **caractérisé en ce que** la couche de rétention est composée de microfibres soufflées par fusion et d'une matière particulaire superabsorbante.
